# EUROPEAN PATENT APPLICATION

(11) **EP 2 042 139 A2**
(43) Date of publication of application: **01.04.2009**
(21) Application number: 08253102.1
(22) Date of filing: 23.09.2008
(51) Int. Cl.: A61F 13/02

(54) **Delivery system for wound dressing**

(30) Priority: 26.09.2007 US 904262
(71) Applicant: Tyco Healthcare Group LP, Mansfield, MA 02048 (US)
(72) Inventor: Vitaris, Ronald F, Worcester, Massachusetts 01606 (US)
(74) Representative: Pratt, David Martin

(57) **Abstract**

A delivery system for applying a wound dressing includes a thin film elastomeric wound dressing having a lower surface for application to the wound area and an upper surface, a release member releasably attached to the lower surface of the wound dressing, a delivery member including a lower surface releasably attached relative to the upper surface of the wound dressing and an upper surface, and being dimensioned to support the wound dressing and thereby facilitate application of the wound dressing to the wound area, and a tab member at least partially disposed between the wound dressing and the delivery member. The wound dressing defines a longitudinal axis and has longitudinal edges along the longitudinal axis and transverse edges traversing the longitudinal axis. The lower surface of the wound dressing may have a pressure sensitive adhesive for securing of the wound dressing relative to the wound area. The tab member is releasably attached relative to the lower surface of the delivery member. The tab member defines a manually engageable section free from releasable attachment to the lower surface of the delivery member and being dimensioned for manual engagement by a clinician to thereby facilitate removal of the tab member from the delivery member.

## Description

### BACKGROUND

### 1. Technical Field

The present invention relates to wound dressings, and more particularly to delivery systems for applying wound dressings to a wound area.

### 2. Discussion of Related Art

The prior art is replete with wound dressings for application to a wound for facilitating healing and closure of the wound. For example, one known wound dressing is POLYSKIN^{®} II, manufactured by Kendall Corporation, a division of TycoHealthcare. POLYSKIN^{®} II is a semi-permeable transparent adhesive film dressing which, when applied to the periwound area, permits moisture transfer while protecting against infection. Delivery of the POLYSKIN^{®} II dressing involves a double tab delivery system which permits one handed aseptic delivery while avoiding folding of the dressing. One suitable delivery system is disclosed in commonly assigned U.S. Patent No. 5,018,516 to Gilman, the entire contents of which disclosure is incorporated herein by reference.

### SUMMARY

Accordingly, the present disclosure is directed to an enhanced delivery system for a wound dressing. A delivery system for applying a wound dressing includes a thin film elastomeric wound dressing having a lower surface for application to the wound area and an upper surface, a release member releasably attached to the lower surface of the wound dressing, a delivery member including a lower surface releasably attached relative to the upper surface of the wound dressing and an upper surface, and being dimensioned to support the wound dressing and thereby facilitate application of the wound dressing to the wound area, and a tab member at least partially disposed between the wound dressing and the delivery member. The wound dressing defines a longitudinal axis and has longitudinal edges along the longitudinal axis and transverse edges traversing the longitudinal axis. The lower surface of the wound dressing may have a pressure sensitive adhesive, on at least a portion of its surface, for securing of the wound dressing relative to the wound area. The tab member is releasably attached relative to the lower surface of the delivery member. The tab member may define a manually engageable section free from releasable attachment to the lower surface of the delivery member and being dimensioned for manual engagement by a clinician to thereby facilitate removal of the tab member from the delivery member.

The tab member may include a fold section having a free lift edge at least partially defining the manually engageable section. The free lift edge is at least partially disposed between transverse edges of the delivery member. In the alternative, the tab member has a linear sheet that covers a selected portion of the tab member and defines a free lift edge. The linear sheet may be coated on the adhesive contacting side with a release agent, such as silicone, and may be removed prior to reattachment. In an alternative embodiment, the free lift edge may be devoid of an adhesive in a targeted area. This adhesive free area may be positioned such that it is overlapping at least one longitudinal edge of the wound dressing between the wound dressing and the delivery film. Alternatively, the free lift edge, defined by the area devoid of adhesive, could be positioned under the delivery film but beyond the periphery of the wound dressing. Further, it is envisioned that the area devoid of adhesive may extend beyond the periphery of both the wound dressing and the delivery film

The delivery film may include at least one window or cut-away area(s). The window(s) may be disposed in a generally central area of the delivery member. Cut-a way's may be disposed generally in the peripheral area. The shape of the window, or windows, is envisioned to be not limited to square or rectangular shapes but to be adaptable and appropriate for the intended end use of the dressing. The wound dressing may define arcuate corners. The release liner includes an upper surface releasably attached to the lower surface of the wound dressing. Suitable liners for use can be made from kraft papers polyethylene, polypropylene, polyesters or composites of any of these materials. The preferred liner material would be either a super calendared Kraft paper or, the more preferred, clay coated Kraft. The upper surface of the release liner has a releasable coating, preferably the release agents would be either Flurochemical or, the preferred, silicones. Examples of a commercially available liner include Converter's Choice® and the preferred 78 Select^{™} produced by Loparex^{®}. The release liner may define a longitudinal length greater than a longitudinal length defined by the wound dressing.

### DESCRIPTION OF THE DRAWINGS

Various embodiments of the subject wound dressing are described herein with reference to the drawings wherein:
FIG. 1 is a perspective view with parts separated of a delivery system in accordance with the principles of the present disclosure illustrating the wound dressing, the release member, the delivery member and the tab member;
FIG. 2 is a top plan view with portions cut away of the delivery system;
FIG. 3 is a cross-sectional view of the delivery system taken along the line 3A-3A of FIG. 2;
FIG. 3A is an enlarged isolated view of the area of detail indicated in FIG. 3;
FIG. 4 is an alternate embodiment of the tab member shown in FIG 3A;
FIG. 5 is an alternative embodiment of the tab member shown in FIG.3A;
FIG. 6 is a fragmentary sectional view illustrating manipulation of the delivery member in order to remove the wound dressing from the release member;
FIG. 7 is a view similar to the view of FIG. 6 illustrating the tab member, with a section devoid of adhesive, extending beyond the periphery of the delivery film to facilitate removal from the wound dressing and/or the delivery member;
FIG. 8 is an alternate embodiment of the invention without a widow or cut-out in the delivery film;
FIG 8A is the embodiment of FIG 8 with a window;
FIG. 9 is an alternative embodiment of FIG 8 with an alternative window configuration containing multiple windows;
FIG. 10 is an alternative embodiment of FIG 8 with an alternative window configuration containing multiple semi-circular cut-outs;
FIG. 11 is an alternative embodiment of FIG 8 with an alternative window configuration containing multiple rectangular cut-outs; and
FIG. 12 is an alternative embodiment of FIG 8 with an alternative window configuration containing multiple triangular cut-outs.

### DETAILED DESCRIPTION OF THE PREFERRED EMBODIMENT(S)

The delivery system of the present disclosure facilitates application of a wound dressing to the periwound area to promote healing of the wound. The delivery system has application for any wound type or condition requiring placement of a wound dressing or film to at least partially cover, enclose the wound and prevent bacteria from communication therewith. The delivery system is non-invasive and substantially eliminates the potential of degradation of the dressing subsequent to application and/or the disruption of the bond established with the periwound area. The delivery system is of simplified construction and reduced cost.

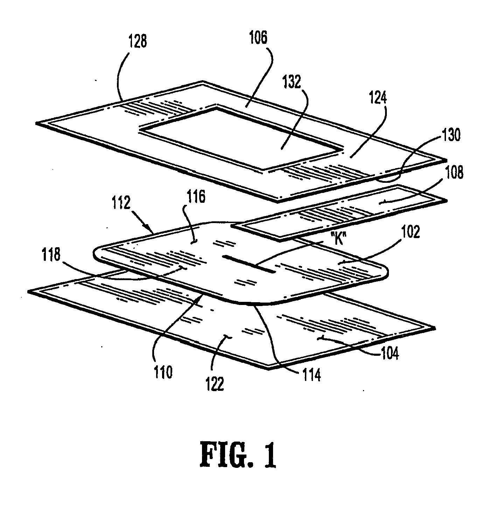

Referring now to FIGS. 1-3A, the delivery system 100 in accordance with one embodiment of the present disclosure is illustrated. Delivery system 100 includes several components, assembled together, namely, wound dressing 102, release member 104, delivery member 106 and tab member 108. Wound dressing 102 may be any occlusive member, dressing, film etc. adapted for positioning over the wound to provide at least a liquid tight enclosure. In one embodiment, wound dressing 102 includes a polyurethane film. Wound dressing 102 may include the transparent dressing manufactured under the trademark POLYSKIN^{®} II by Kendall Corp, a division of TycoHealthcare. POLYSKIN^{®} II is a transparent, semi-permeable material which permits moisture and oxygen exchange with the wound site, and provides a barrier to microbes and fluid containment. The transparency of wound dressing 102 provides a visual indication of the status of the healing process of the wound area. In the alternative, wound dressing 102 may include an impermeable material.

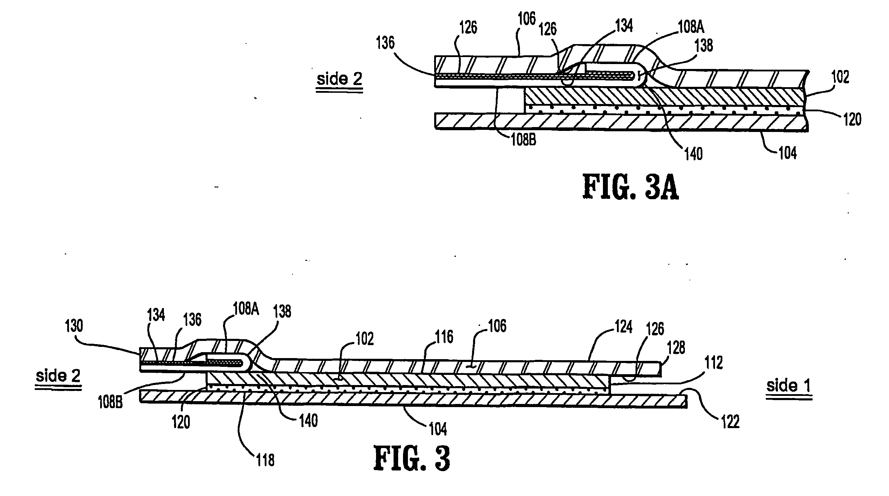

For reference purposes, wound dressing 102 defines longitudinal axis "k" extending along the length of the wound dressing 102 and has longitudinal edges 110 and side or transverse edges 112 which traverse the longitudinal axis "k". Although shown as being generally rectangular in shape, wound dressing 102 may assume other configurations including, but, not limited to, triangular, elliptical, round, square or any other shape contemplated by the clinician. Wound dressing 102 further includes generally arcuate-shaped or rounded corners 114 interconnecting longitudinal and side edges 110, 112. Corners 114 may define a right angle if desired. Wound dressing 102 further includes upper and lower surfaces 116, 118. Lower surface 118 is adjacent the wound when applied to the patient. Lower surface 118 may further include pressure sensitive adhesive material 120 such as an acrylic or rubber based adhesive adapted to secure wound dressing 102 relative to the wound area (FIG. 3). Adhesive material 120 may be disposed adjacent the peripheral margins or areas of wound dressing 102 or may encompass a major portion of lower surface 118.

Release member 104 is a release sheet or liner releasably attached to and preferably covering adhesive material 120 of wound dressing 102. Release member 104 is preferably removed from wound dressing 102 immediately prior to application to the periwound area to maintain the effectiveness of the adhesive material 120 and prevent inadvertent sticking of the adhesive material 120 to undesired surfaces. Release member 104 may assume a suitable form known in the art such as a release liner, preferably a clay coated kraft, having a silicone release coating which faces adhesive material 120 of wound dressing 102. In one embodiment, release member 104 includes upper surface 122 releasably attached to lower surface 118, e.g., adhesive material 120 of wound dressing 120, with the upper surface 122 of the release member 104 having a releasable coating such as silicone. Release member 104 may define a longitudinal length greater than a longitudinal length defined by the wound dressing 102.

Delivery member 106 is intended to function as a support sheet for wound dressing 102 during packaging, transport and application of the wound dressing 102 to the patient. Delivery member 106 may be constructed from any suitable material which would be more stiff or rigid than wound dressing 102, such as a relatively stiff plastic film, e.g. ethylene vinyl acetate, polyethylene, polyethylene, polyester or Metallocene. Delivery member has upper surface 124, lower surface 126 and side edges 128,130. Delivery member 106 may further include cut-out or window 132 located within a central area of the delivery member 106. Window 132 provides additional flexibility to facilitate application to irregular surfaces or topographies of the skin such as the foot, groin, or base of the neck. Delivery member 106 may be also devoid of window.

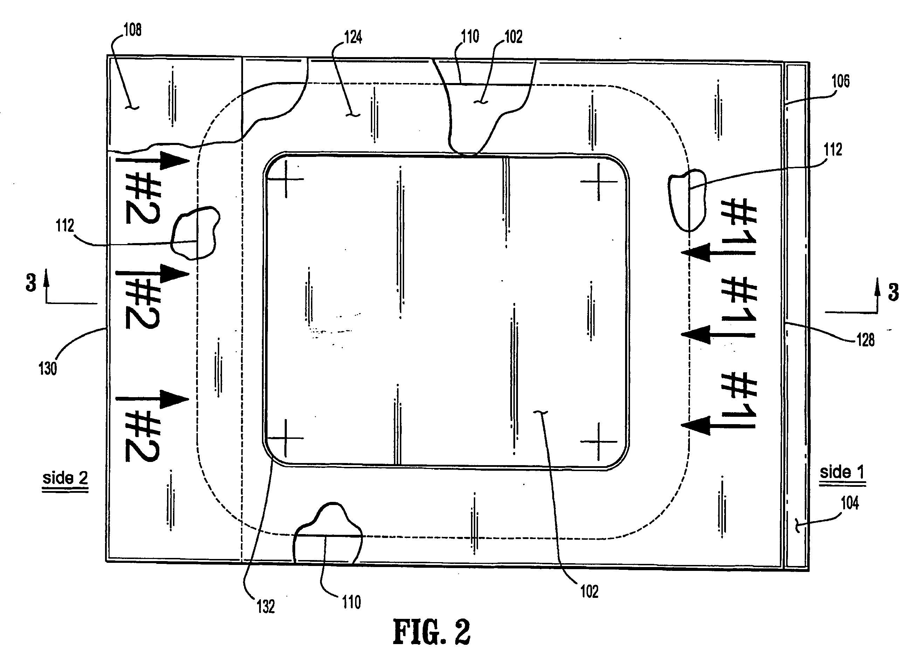

Tab member 108 is disposed between wound dressing 102 and delivery film 106 along a portion of its length. In one embodiment, tab member 108 includes upper surface 134 having adhesive material 136 covering at least part of or the entire surface 134 i, to permit the tab member 108 to be releasably attached to lower surface 126 of delivery member 106. When assembled within delivery system 100, tab member 108 is folded onto itself to define fold section 138 as depicted in FIGS. 3 and 3A. Fold section 138 includes defines upper segment 108a of tab member 108, which has adhesive material 136 to releasably secure tab member 108 to delivery member 106. Lower segment 108b of tab member 108 defined by fold section 138 is free of adhesive material and thus is not bound to underlying wound dressing 102. Thus, the upper and lower segments of tab member 108, created by fold section 138, define a lift edge 140 which, in conjunction with fold section 138, is dimensioned for manual engagement by a clinician to facilitate removal of the tab member 108 from the delivery member 106 subsequent to application of delivery system 100. Free lift edge 140 may be disposed inward or between transverse edges 112 of wound dressing 102. Preferably, as best depicted in FIG. 2, tab member 108 extends beyond both longitudinal edge 110 and side edge 112 of wound dressing 102.

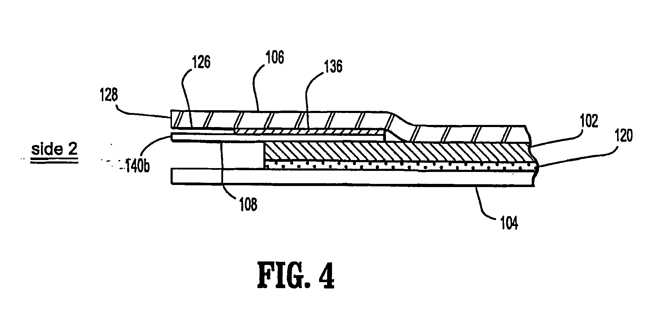

In the alternative, tab member 108 may be devoid of adhesive 138 in selected areas. As shown in FIG.4, the adhesive area devoid of adhesive produces a lift edge 140b by not allowing the entire surface of tab 108 to be attached to the surface 126 of delivery film 106. FIG.4 show the lift edge in-board of the periphery of dressing 102 on side 112, in-between dressing 102 and delivery film 106. In an alternative configuration the lift edge of tab 108 formed by and area devoid of adhesive may extend beyond the side edge of delivery film 106. Additionally in this alternative configuration, tab 108 extends inboard of side edge 112. In an additional alternative, the tab member 108 may not extend beyond the periphery of delivery film 106, The lift edge formed by the area devoid of adhesive would be positioned inboard of side edge 112, in between the delivery film 106 and the wound dressing 102 Tab member 108, once removed from the delivery film after the dressing is applied to the patient. may provide a surface on which legends pertaining to patient information, dressing status etc. can be written by the clinician. Additional, when the non-windowed embodiment of this invention is used, the delivery film, because of its relative clarity, may be used as to trace the wound margins, providing the clinician with another means of documenting wound change.

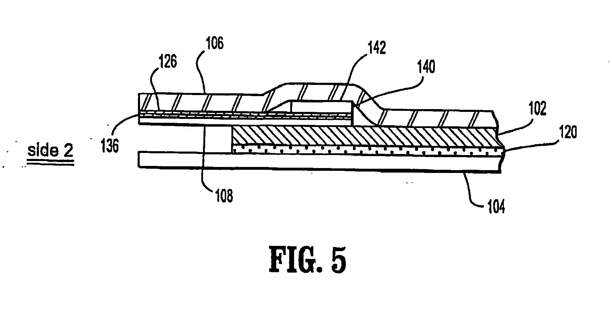

In an alternate embodiment depicted in FIG 5, tab member 108 is devoid of a fold section, but, rather includes a linear sheet. Tab member 108 includes manually engageable section 142 which extends beyond longitudinal edge 110 of wound dressing 102. Preferably, the upper surface of tab member 108 overlapping lower surface 126 of delivery member 106 includes an adhesive material 136 to secure the tab member 108 to the delivery member 106. An alternative embodiment to the FIG 5 (not illustrated herein) would be to extend the releasable linear sheet of FIG 5 to cover the entirety of adhesive 136 with the release coated side of the linear sheet 142. Securement to the lower surface of the delivery film would be accomplished by another layer of adhesive that would adhere to the non release side of 142 to the lower side 126 of delivery film 106.

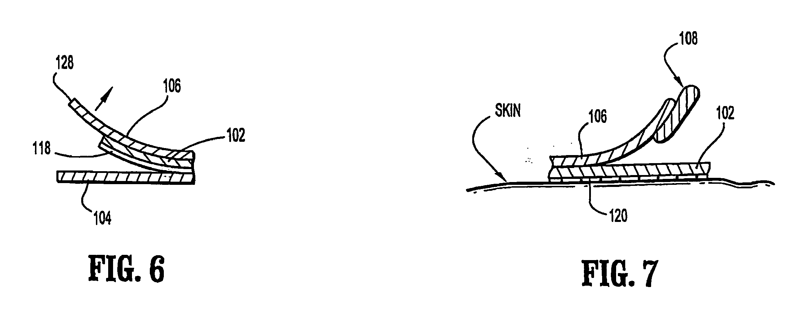

Referring now to FIG 6, in use, delivery member 106 is grasped with one hand adjacent side edge 128 and release member 104 is grasped with the other hand, and the release member 104 is removed from wound dressing 102 as shown by the directional arrow. In this regard, release member 104 is removed in a simplified manner from wound dressing 102 to render the wound dressing 102 in condition for application to the wound of a patient. Thereafter, wound dressing 102 is applied to the wound area with delivery member 106 providing the necessary support for the wound dressing 102 during application. During application, tab member 108, which is stiffer than wound dressing 102, provides rigidity and support for application of the dressing end margin adjacent side edge 112 of the wound dressing 102.

After application of wound dressing 102 on the patient, delivery member 106 is grasped adjacent tab member 108 in order to peel the delivery member 106 from wound dressing 102 as depicted in FIG 7 and shown by the directional arrow. Regardless of the methodology incorporated, in that tab member 108 is not releasably attached or bonded to wound dressing 102, the tab member 108 may be removed relative to the wound dressing 102 with substantially no effect on the wound dressing 102 or the adhesive bond created between the wound dressing 102 and the patient's wound area.

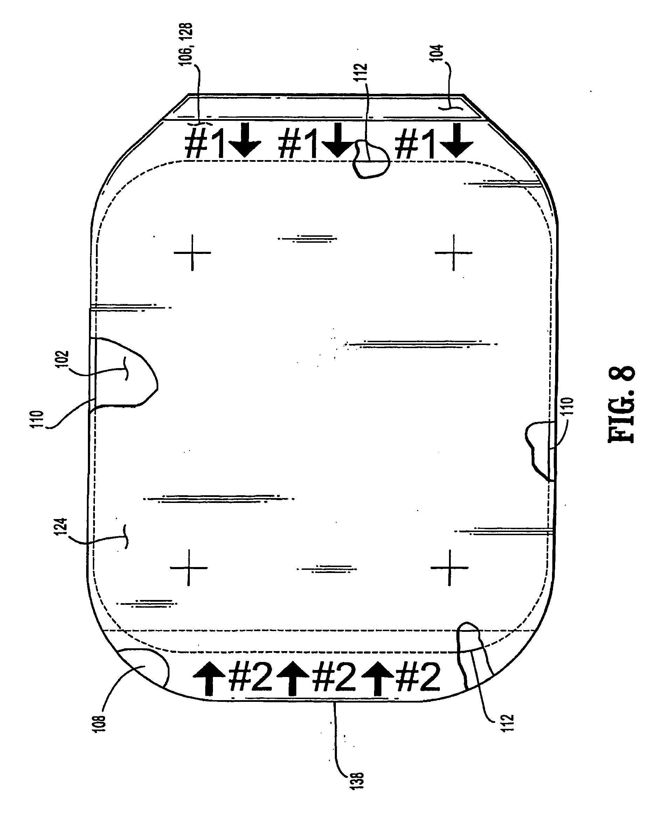

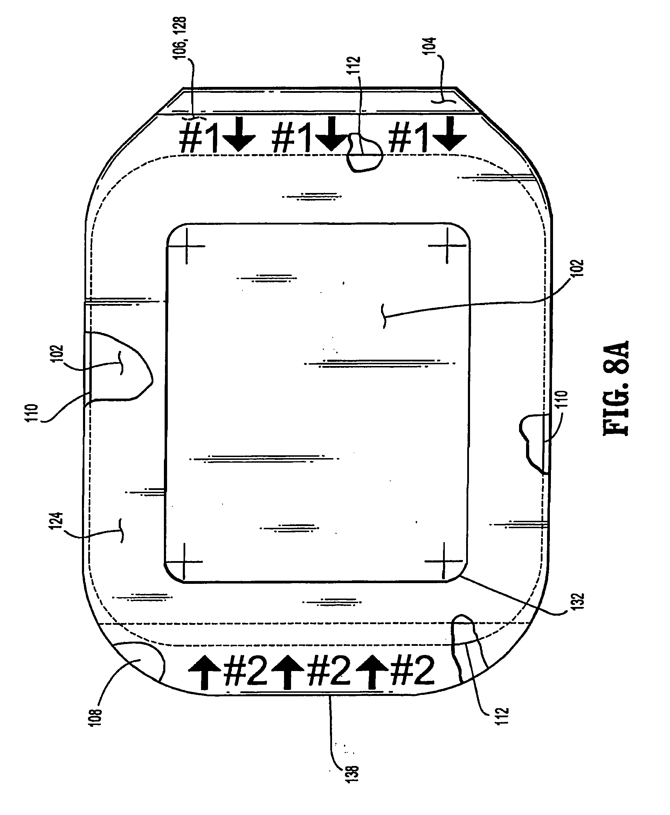

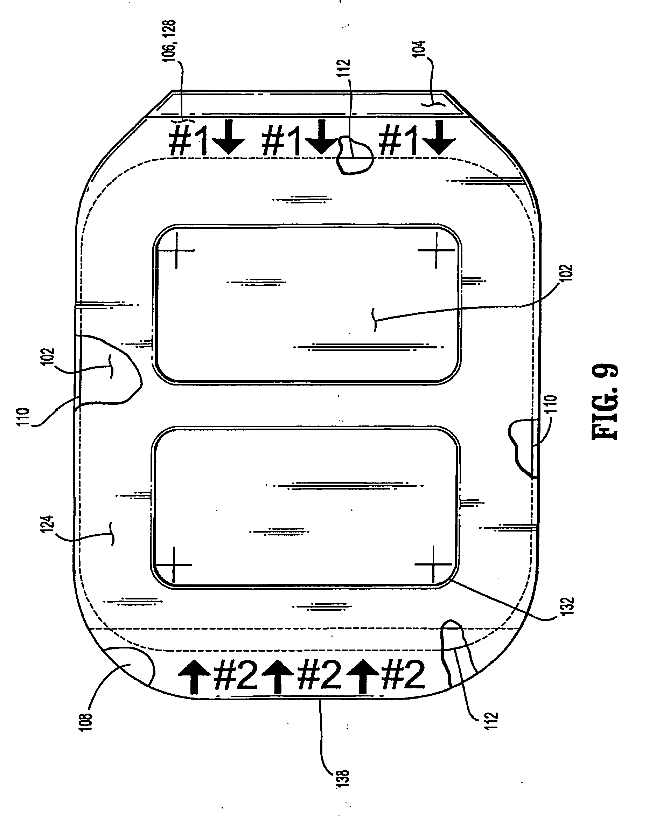

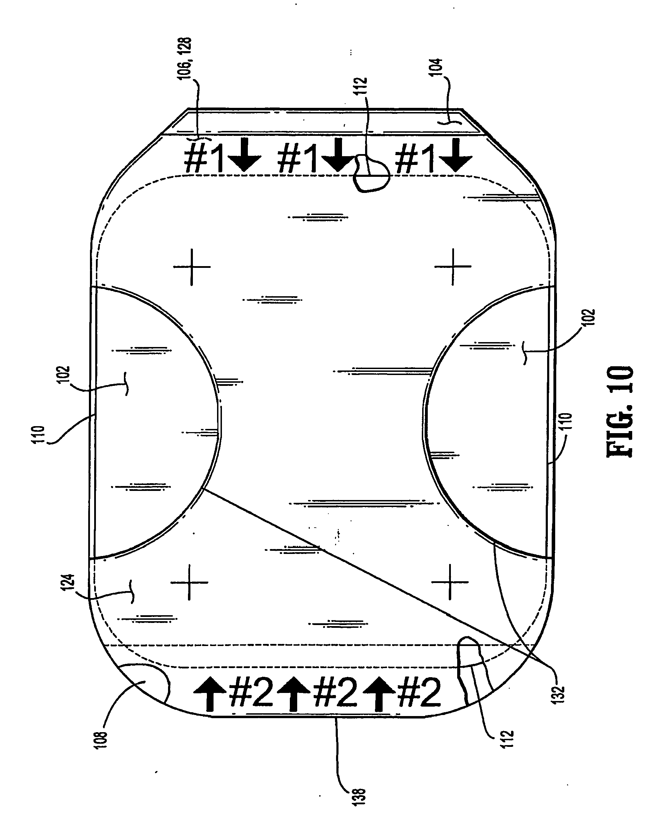

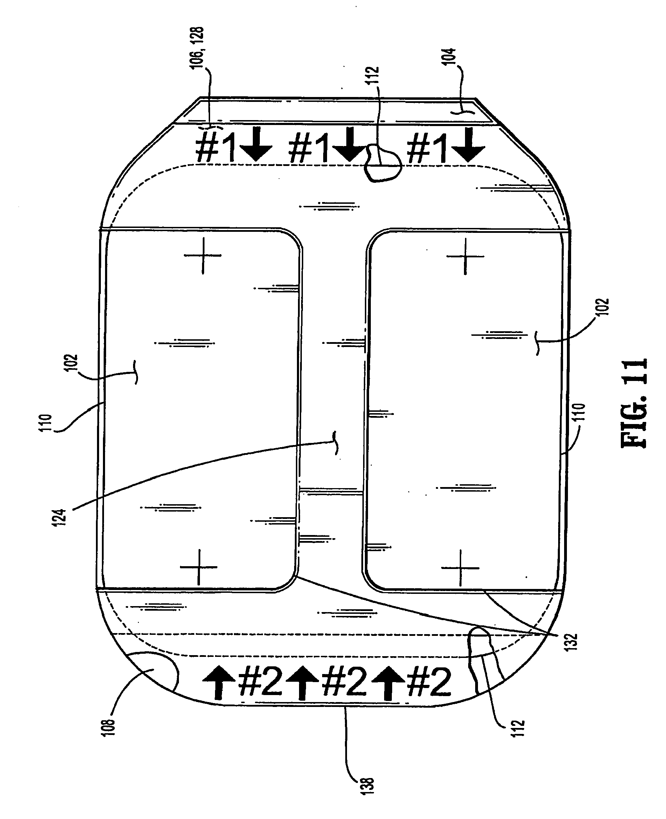

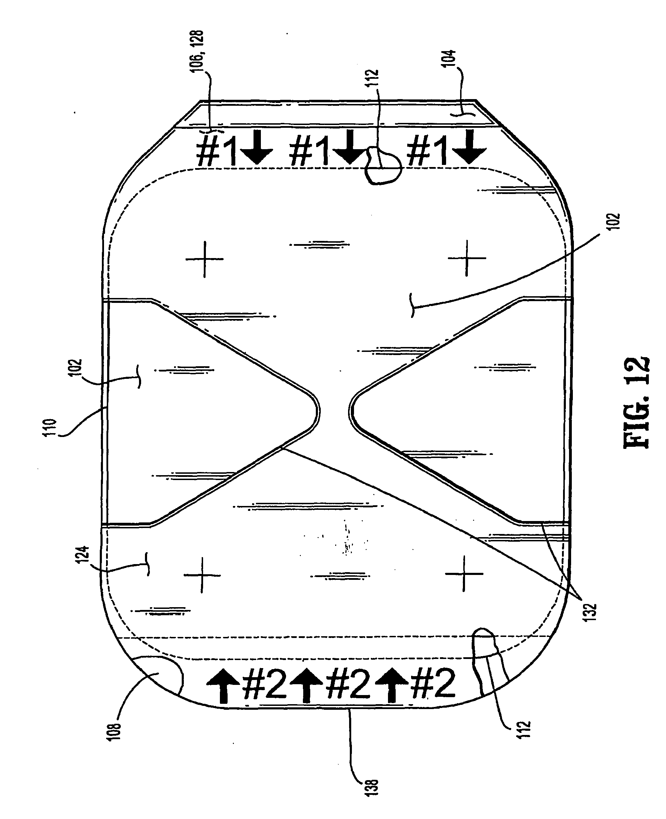

FIGS. 8-12 illustrate additional embodiments of the disclosure. In FIG. 8, delivery member 106 is devoid of window 132. In FIG. 8A, delivery member 106 has window 132. FIG. 9 illustrates delivery member 106 with a plurality, e.g., two, windows 132. FIG. 10 illustrates delivery member 106 with window 132 in the general shape of a semi-circle. FIG. 11 illustrates delivery member 106 with multiple windows 132 in the general shape of a rectangle while FIG. 12 illustrating generally triangular windows 132. Other embodiments and configurations are also envisioned.

While the disclosure has been illustrated and described, it is not intended to be limited to the details shown, since various modifications and substitutions can be made without departing in any way from the spirit of the present disclosure. Accordingly, further modifications and equivalents of the invention herein disclosed can occur to persons skilled in the art, and all such modifications and equivalents are believed to be within the spirit and scope of the disclosure as defined by the following claims

## Claims

1. A delivery system for applying a wound dressing, which comprises:
a thin film elastomeric wound dressing having a lower surface for application to the wound area and an upper surface, the wound dressing defining a longitudinal axis and having longitudinal edges along the longitudinal axis and transverse edges traversing the longitudinal axis, the lower surface having a pressure sensitive adhesive for securing of the wound dressing relative to the wound area;
a release member releasably attached to the lower surface of the wound dressing;
a delivery member including a lower surface releasably attached relative to the upper surface of the wound dressing and an upper surface, the delivery member dimensioned to support the wound dressing and thereby facilitate application of the wound dressing to the wound area; and
a tab member at least partially disposed between the wound dressing and the delivery member, the tab member releasably attached relative to the lower surface of the delivery member, the tab member defining a manually engageable section free from releasable attachment to the lower surface of the delivery member and dimensioned for manual engagement by a clinician to thereby facilitate removal of the tab member from the delivery member.

2. The delivery system according to claim 1 wherein the tab member includes a fold section, the fold section having a free lift edge at least partially defining the manually engageable section.

3. The delivery system according to claim 2 wherein the free lift edge is at least partially disposed between transverse edges of the delivery member.

4. The delivery system according to claim 3 wherein the free lift edge extends beyond at least one longitudinal edge of the delivery member.

5. The delivery system according to claim 1 wherein the tab member defines a free lift edge, the free lift edge extending beyond one of a longitudinal edge and a side edge of the delivery member.

6. The delivery system according to claim 5 wherein the free lift edge is devoid of an adhesive.

7. The delivery system according to claim 1 wherein the delivery member includes a window.

8. The delivery system according to claim 7 wherein the window is disposed in a generally central area of the delivery member.

9. The delivery system according to claim 7 wherein the delivery member includes a plurality of windows.

10. The delivery system according to claim 1 wherein the wound dressing defines arcuate corners.

11. The delivery system according to claim 1 wherein the release paper includes an upper surface releasably attached to the lower surface of the wound dressing, the upper surface of the release paper having a releasable coating.

12. The delivery system according to claim 11 wherein the releasable coating comprises Loparex.

13. The delivery system according to claim 1 wherein the release paper defines a longitudinal length greater than a longitudinal length defined by the wound dressing.
